# EUROPEAN PATENT APPLICATION

(11) **EP 3 733 678 A1**
(43) Date of publication of application: **04.11.2020**
(21) Application number: 18897718.5
(22) Date of filing: 27.12.2018
(51) Int. Cl.: C07F 3/02, C07C 1/32, C07C 15/27, C07F 5/02, C07B 61/00

(54) **METHOD FOR SYNTHESIZING ORGANIC MAGNESIUM COMPOUND, METHOD FOR SYNTHESIZING ORGANIC BORIC ACID COMPOUND, AND COUPLING METHOD**

(30) Priority: 27.12.2017 JP 2017252323
(71) Applicant: Kobelco Eco-Solutions Co., Ltd, Kobe-shi, Hyogo 651-0072 (JP); National University Corporation Okayama University, Kita-ku Okayama-shi Okayama 700-8530 (JP)
(72) Inventor: MURAKAMI Yoshiaki, Kobe-shi, Hyogo 651-2241 (JP); FUKUSHIMA Miyuki, Kobe-shi, Hyogo 651-2241 (JP); TAKAI Kazuhiko, Okayama-shi, Okayama 700-8530 (JP); ASAKO Sobi, Okayama-shi, Okayama 700-8530 (JP)
(74) Representative: Winter, Brandl, Fürniss, Hübner, Röss, Kaiser, Polte - Partnerschaft mbB
(86) International application number: PCT/JP2018/048213
(87) International publication number: WO 2019/131898

(57) **Abstract**

An object is to establish a technology with which an organic magnesium compound can be easily and efficiently synthesized at a low cost with few steps that do not involve a complex chemical method. A method for synthesizing an organic magnesium compound includes, in a reaction solvent, reacting an organic halide represented by General Formula I (R^{a}-X^{a}) with a dispersion product obtained by dispersing sodium in a dispersion solvent to obtain an organic sodium compound represented by General Formula II (R^{a}-Na), and reacting the obtained organic sodium compound with a magnesium halide represented by General Formula III (Mg-(X^{b})₂) to obtain an organic magnesium compound represented by General Formula IV (R^{a}-Mg-X^{b}).

## Description

### Technical Field

The present invention relates to a method for synthesizing an organic magnesium compound, an organic boronic acid compound obtained using an organic magnesium compound, and a coupling method.

### Background Art

Grignard reagents, which are representative examples of organic magnesium compounds, are organic magnesium halides and widely used as carbon nucleophilic agents for carbon-carbon bond forming reactions and the like. Also, it is known that organic boronic acid compounds that are organic boron compounds used in Suzuki-Miyaura cross coupling, in which coupling is performed by forming carbon-carbon bonds in the presence of a palladium catalyst, can be synthesized using Grignard reagents. As described above, Grignard reagents are used as important reactants and raw materials in natural product total synthesis and organic synthesis reactions of various functional materials such as medicines, agricultural chemicals, electronic materials, and intermediate products thereof.

Grignard reagents are commonly obtained by reacting an organic halide with magnesium metal in an anhydrous solvent such as tetrahydrofuran (hereinafter referred to as "THF") or an ether. As methods for synthesizing organic boronic acid compounds, which are organic boron compounds, a method for reacting an organometallic compound with a borate ester compound and a method for reacting an organic halide with diboronic acid or the like in the presence of a palladium catalyst are known, in addition to the above-described method in which a Grignard reagent is used. Further, it has been reported that an aromatic boronic acid compound can be directly synthesized through activation of carbon-hydrogen bonds by using an iridium catalyst and an electron donating bidentate ligand, but it is also known that substituents on an aromatic ring have a strong three-dimensional influence on this method. On the other hand, it has been reported that triphenylboron is synthesized as an organic boronic acid compound through a reaction between sodium metal, an aromatic halide (chlorobenzene), and a secondary alkyl borate ester compound (see Non-Patent Document 1, for example). Also, it has been reported that triphenylboron is synthesized from sodium sand, chlorobenzene, and triisopropyl borate (TIPB), using toluene as a solvent (see Non-Patent Document 2, for example).

### Prior Art Documents

### Non-Patent Documents

Non-Patent Document 1: C. R. Guibert and J. L. Little, "Alkyl- and Arylboranes," Ullmanns's Encyclopedia of Industrial Chemistry, Wiley-VCH Verlag, Weinheim, 2005
Non-Patent Document 2: Xu Jiantao et al., "Preparation of Triphenylboron" Shiyou Huagong, 2013 42(4), 415-418

### Disclosure of the Invention

### Problem to be Solved by the Invention

Generally, organic bromides (R-Br) and organic iodides (R-I) have higher reactivity than organic chlorides (R-Cl) in the synthesis of a Grignard reagent, but organic bromides and organic iodides are expensive. On the other hand, organic chlorides are inexpensive when compared to other organic halides, but have low reactivity in the synthesis, and accordingly are likely to reduce the yield of a Grignard reagent to be obtained. In particular, in the synthesis of an aromatic Grignard reagent, an alkenyl Grignard reagent, and the like, the Grignard reagent to be obtained cannot be synthesized with a high yield, and it is also known that the yield is affected by substituents on an aromatic ring and the like. Therefore, conventionally known methods for synthesizing a Grignard reagent cannot sufficiently satisfy market requirements from industrial and economic standpoints. Also, there is a problem in that in the preparation of a Grignard reagent, such as an aromatic Grignard reagent, which is likely to cause Wurtz coupling, strict temperature control and the like need to be performed to suppress the occurrence of Wurtz coupling that causes a reduction in the yield.

Therefore, there has been demand for establishing a technology with which an organic magnesium compound can be easily and efficiently synthesized at a low cost with few steps that do not involve a complex chemical method.

### Means for Solving Problem

Inventors of the present invention conducted studies to solve the above-described problems, and found that an organic magnesium compound that can be used as a Grignard reagent can be efficiently synthesized by reacting, in a reaction solvent, an organic halide with a dispersion product obtained by dispersing sodium in a dispersion solvent to obtain an organic sodium compound, and then reacting the obtained organic sodium compound with a magnesium halide. In the synthesis method, a dispersion product that is obtained by dispersing sodium in a dispersion solvent and can be easily handled is used, and accordingly, an organic magnesium compound can be synthesized under moderate conditions. Furthermore, the organic magnesium compound can be synthesized at a low cost with few steps that do not involve a complex chemical method. The inventors of the present invention completed the present invention based on these findings.

That is, the present invention relates to a method for synthesizing an organic magnesium compound and has a characteristic configuration in which the method includes, in a reaction solvent, reacting an organic halide represented by General Formula I (R^{a}-X^{a}), where R^{a} is an aliphatic hydrocarbon group, an alicyclic hydrocarbon group, or an aromatic hydrocarbon group, which optionally includes a substituent that does not react with sodium, and X^{a} is a halogen atom, with a dispersion product obtained by dispersing sodium in a dispersion solvent to obtain an organic sodium compound represented by General Formula II (R^{a}-Na), where R^{a} is the same as R^{a} in the General Formula I, and
reacting the obtained organic sodium compound with a magnesium halide represented by General Formula III (Mg-(X^{b})₂), where X^{b} is a halogen atom, to obtain an organic magnesium compound represented by General Formula IV (R^{a}-Mg-X^{b}), where R^{a} is the same as R^{a} in the General Formula I and X^{b} is the same as X^{b} in the General Formula III or R^{a}.

According to this configuration, it is possible to provide a method for synthesizing an organic magnesium compound with which an organic magnesium compound can be stably and efficiently synthesized from an organic halide through the synthesis of an organic sodium compound. In this configuration, the dispersion product that is obtained by dispersing sodium in a dispersion solvent and can be easily handled is used, and accordingly, the organic magnesium compound can be easily synthesized under moderate conditions at a low cost in a short time with few steps that do not involve a complex chemical method, and therefore the synthesis method is extremely advantageous from economic and industrial standpoints. Furthermore, the reaction progresses under moderate conditions, and therefore the organic sodium compound can be efficiently obtained without the occurrence of a side reaction such as a Wurtz reaction in which organic halides are coupled to each other, and consequently the organic magnesium compound can be synthesized with a high yield and high purity. Furthermore, sodium is extremely widely distributed on the earth, and therefore this technology has excellent sustainability. The method for synthesizing an organic magnesium compound according to the present embodiment can be favorably used for the preparation of an aromatic Grignard reagent, in particular, an aromatic Grignard reagent including a bulky substituent, and an alkenyl Grignard reagent, which have been difficult to prepare. Furthermore, the organic magnesium compound synthesized using the method for synthesizing an organic magnesium compound according to this configuration can be favorably used for the synthesis of an organic boronic acid compound that can be used for Suzuki-Miyaura coupling, Kumada-Tamao-Corriu coupling, and the like. Accordingly, the method for synthesizing an organic magnesium compound according to this configuration can be used in various technical fields such as natural product total synthesis and the synthesis of functional materials such as medicines, agricultural chemicals, and electronic materials.

Another characteristic configuration is a method for synthesizing an organic boronic acid compound, including reacting the organic magnesium compound according to the above-described [1] with a borate ester compound represented by General Formula V (B-(O-R^{b})₃), where R^{b} is an aliphatic hydrocarbon group, an alicyclic hydrocarbon group, or an aromatic hydrocarbon group, which optionally includes a substituent, to obtain an organic boronic acid compound represented by General Formula VI (R^{a}-B(OH)₂), where R^{a} is the same as R^{a} in the General Formula I.

According to this characteristic configuration, an organic boronic acid compound that can be favorably used for Suzuki-Miyaura cross coupling and the like can be efficiently synthesized using an organic magnesium compound synthesized using a dispersion product obtained by dispersing sodium in a dispersion solvent. Accordingly, the method for synthesizing an organic boronic acid compound according to this configuration can be used in various technical fields such as natural product total synthesis and the synthesis of functional materials such as medicines, agricultural chemicals, and electronic materials.

Another characteristic configuration is a coupling method including reacting the organic magnesium compound according to the above-described [1] with an organic halide represented by General Formula VIII (R^{c}-X^{c}), where R^{c} is an aromatic hydrocarbon group or an allyl group, which optionally includes a substituent, and X^{c} is a halogen atom, in the presence of an iron catalyst, a nickel catalyst, a palladium catalyst, a chromium catalyst, a manganese catalyst, a cobalt catalyst, or a copper catalyst to obtain a coupling compound represented by General Formula IX (R^{a}-R^{c}), where R^{a} is the same as R^{a} in the General Formula I and R^{c} is the same as R^{c} in the General Formula VIII.

According to this characteristic configuration, it is possible to provide a coupling method in which an organic magnesium compound synthesized using a dispersion product obtained by dispersing sodium in a dispersion solvent is used for Kumada-Tamao-Corriu coupling. Accordingly, the coupling method according to this configuration can be used in various technical fields such as natural product total synthesis and the synthesis of functional materials such as medicines, agricultural chemicals, and electronic materials.

### Brief Description of the Drawings

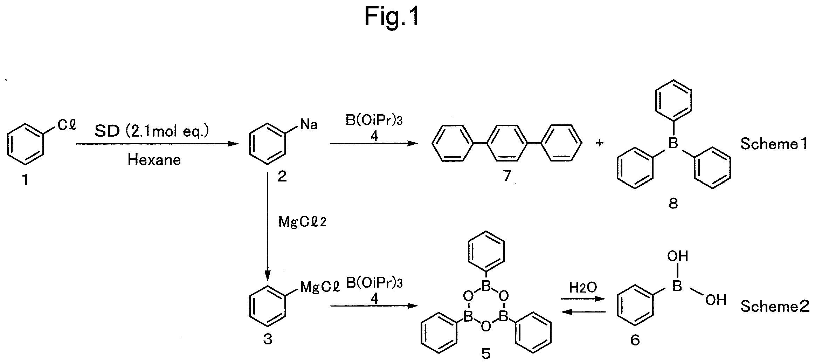

FIG. 1 is a diagram showing a summary of synthesis conditions and results of Example 1 and Preliminary Investigation Example 1 in which a method for synthesizing an organic magnesium compound and a method for synthesizing an organic boronic acid compound using an organic magnesium compound according to an embodiment of the present invention were investigated.

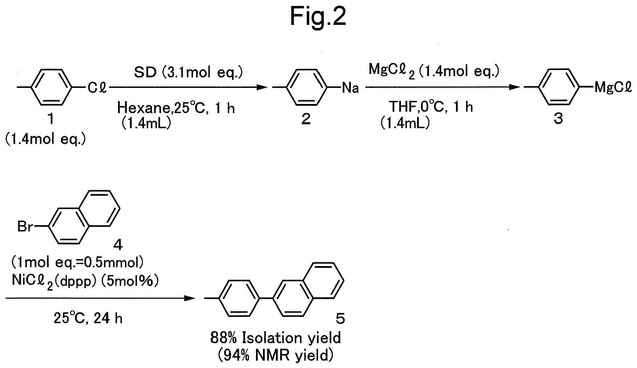

FIG. 2 is a diagram showing a summary of synthesis conditions and results of Example 2 in which conditions for synthesizing an organic magnesium compound according to an embodiment of the present invention and application to Kumada-Tamao-Corriu coupling were investigated.

### Best Mode for Carrying out the Invention

The following describes a method for synthesizing an organic magnesium compound, a method for synthesizing an organic boronic acid compound using an organic magnesium compound, and a coupling method according to an embodiment of the present invention in detail. However, the present invention is not limited to the embodiment described below.

### Method for Synthesizing Organic Magnesium Compound

The method for synthesizing an organic magnesium compound according to the present embodiment is a method for synthesizing an organic magnesium halide that can be used as a Grignard reagent. In a reaction solvent, an organic halide represented by General Formula I (R^{a}-X^{a}) is reacted with a dispersion product obtained by dispersing sodium in a dispersion solvent to obtain an organic sodium compound represented by General Formula II (R^{a}-Na) (step 1). Subsequently, the obtained organic sodium compound is reacted with a magnesium halide represented by General Formula III (Mg-(X^{b})₂) to obtain an organic magnesium compound represented by General Formula IV (R^{a}-Mg-X^{b}) (step 2).

### Step 1

As expressed by Reaction Formula I (R^{a}-X^{a} + 2Na (as SD) → R^{a}-Na + Na-X^{a}), step 1 is a step for reacting, in a reaction solvent, an organic halide represented by General Formula I (R^{a}-X^{a}) with a dispersion product obtained by dispersing sodium in a dispersion solvent to obtain an organic sodium compound represented by General Formula II (R^{a}-Na). The organic halide represented by General Formula I (R^{a}-X^{a}) is an organic compound that includes a covalently bonded halogen atom and is a starting compound of the method for synthesizing an organic magnesium compound according to the present embodiment. The method for synthesizing an organic magnesium compound according to the present embodiment can be favorably used for the preparation of an aromatic Grignard reagent, in particular, an aromatic Grignard reagent including a bulky substituent, and an alkenyl Grignard reagent, which have been difficult to prepare, and accordingly, an aromatic or alkenyl halide can be used as the starting compound.

In the organic halide represented by General Formula I (R^{a}-X^{a}), R^{a} is an aliphatic hydrocarbon group, an alicyclic hydrocarbon group, or an aromatic hydrocarbon group, which optionally includes a substituent that does not react with sodium. It is not preferable to include a substituent that reacts with sodium because such a substituent causes a side reaction by reacting with the dispersion product obtained by dispersing sodium in a dispersion solvent. Therefore, if the starting compound is a compound that includes, as R^{a}, a substituent that reacts with sodium, the substituent needs to be protected by an appropriate protective group or the like.

The aliphatic hydrocarbon group may be linear or branched, and saturated or unsaturated. There is no particular limitation on the chain length. If a substituent is included, there is no particular limitation on the substituent so long as the substituent does not react with sodium. Also, there is no particular limitation on the number of substituents and the position to which a substituent is introduced. Examples of the aliphatic hydrocarbon group include, but are not limited to, alkyl groups, alkenyl groups, and alkynyl groups that preferably have 1 to 50 carbon atoms or 1 to 30 carbon atoms, and particularly preferably have 3 to 20 carbon atoms. Specifically, examples of alkyl groups include, but are not limited to, methyl group, ethyl group, propyl group, butyl group, methyl group, ethyl group, propyl group, isopropyl group, n-butyl group, isobutyl group, s-butyl group, t-butyl group, n-pentyl group, isopentyl group, neopentyl group, t-pentyl group, s-pentyl group, 2-methylbutyl group, 1-ethylpropyl group, 2-ethylpropyl group, n-hexyl group, isohexyl group, neohexyl group, t-hexyl group, 2,2-dimethylbutyl group, 2-methylpentyl group, 3-methylpentyl group, 1-ethylbutyl group, 2-ethylbutyl group, 1-propylpropyl group, n-heptyl group, isoheptyl group, s-heptyl group, t-heptyl group, 2,2-dimethylpentyl group, 3,3-dimethylpentyl group, 1-methylhexyl group, 2-methylhexyl group, 3-methylhexyl group, 4-methylhexyl group, 1-ethylpentyl group, 2-ethylpentyl group, 3-ethylpentyl group, 1-propylbutyl group, 2-propylbutyl group, n-octyl group, isooctyl group, t-octyl group, neooctyl group, 2,2-dimethylhexyl group, 3,3-dimethylhexyl group, 4,4-dimethylhexyl group, 1-methylheptyl group, 2-methylheptyl group, 3-methylheptyl group, 4-methylheptyl group, 5-methylheptyl group, 1-ethylhexyl group, 2-ethylhexyl group, 3-ethylhexyl group, 4-ethylhexyl group, 1-propylpentyl group, 2-propylpentyl group, 3-propylpentyl group, n-nonyl group, isononyl group, t-nonyl group, 1-methyloctyl group, 2-methyloctyl group, 3-methyloctyl group, 4-methyloctyl group, 5-methyloctyl group, 6-methyloctyl group, n-decyl group, isodecyl group, t-decyl group, 1-methylnonyl group, 2-methylnonyl group, 3-methylnonyl group, 4-methylnonyl group, 5-methylnonyl group, 6-methylnonyl group, 7-methylnonyl group. Examples of alkenyl groups include, but are not limited to, ethenyl group, propenyl group, butenyl group, pentenyl group, hexenyl group, heptenyl group, octenyl group. Examples of alkynyl groups include, but are not limited to, ethynyl group, propynyl group, butynyl group, pentynyl group, heptynyl group, octynyl group.

The aliphatic hydrocarbon group optionally includes a substituent. The aliphatic hydrocarbon group may include one or more substituents, and if a plurality of substituents are included, the substituents may be the same as or differ from each other. Examples of substituents include, but are not limited to, an aliphatic hydrocarbon group, an alicyclic hydrocarbon group, and an aromatic hydrocarbon group, which optionally include substituents. Note that examples of the aliphatic hydrocarbon group are the same as those described above, and examples of the alicyclic hydrocarbon group and the aromatic hydrocarbon group are the same as those described below.

As for the alicyclic hydrocarbon group, bonds between ring constituent atoms may be saturated or unsaturated, and there is no particular limitation on the number of ring members. Also, examples of the alicyclic hydrocarbon group include not only alicyclic hydrocarbon groups that have a single ring but also alicyclic hydrocarbon groups that have a group of rings, such as fused rings and spiro rings. Examples of the alicyclic hydrocarbon group include, but are not limited to, cycloalkyl groups that preferably have 3 to 10 carbon atoms and particularly preferably have 3 to 7 carbon atoms, and cycloalkenyl groups that preferably have 4 to 10 carbon atoms and particularly preferably have 4 to 7 carbon atoms. Specific examples of cycloalkyl groups include, but are not limited to, cyclopropyl group, cyclobutyl group, cyclopentyl group, cyclohexyl group, cycloheptyl group, cyclooctyl group. Examples of cycloalkenyl groups include, but are not limited to, cyclopropenyl group, cyclobutenyl group, cyclopentenyl group, cyclohexenyl group, cycloheptenyl group, cyclooctenyl group.

The alicyclic hydrocarbon group optionally includes a substituent. The alicyclic hydrocarbon group may include one or more substituents, and if a plurality of substituents are included, the substituents may be the same as or differ from each other. There is no particular limitation on positions of substituents. Examples of substituents are the same as those described as examples of substituents of the aliphatic hydrocarbon group.

There is no particular limitation on the aromatic hydrocarbon group so long as the aromatic hydrocarbon group includes an aromatic ring. Examples of the aromatic hydrocarbon group include not only aromatic hydrocarbon groups that have a single ring but also aromatic hydrocarbon groups that have a group of rings, such as fused rings and spiro rings. There is no particular limitation on the number of ring members. The number of carbon atoms included in the aromatic hydrocarbon group is preferably 6 to 22, and particularly preferably 6 to 14, for example. Examples of the aromatic hydrocarbon group include, but are not limited to: a six-membered ring such as monocyclic phenyl group, a two-membered ring such as naphtyl group, pentalenyl group, indenyl group, azulenyl group, etc., a three-membered ring such as biphenylenyl group, indacenyl group, acenaphthylenyl group, fluorenyl group, phenalenyl group, phenanthrenyl group, anthryl group, etc., a four-membered ring such as fluoranthenyl, aceanthryleneyl group, triphenylenyl group, pyrenyl group, naphthacenyl group, etc., a five-membered ring such as perylenyl group, tetraphenylenyl, etc., a six-membered ring such as pentacenyl group, etc., and a seven-membered ring such as rubicenyl group, coronenyl group, heptacenyl group, etc. Particularly preferably, the aromatic hydrocarbon group is a phenyl group.

The aromatic hydrocarbon group optionally includes a substituent. The aromatic hydrocarbon group may include one or more substituents, and if a plurality of substituents are included, the substituents may be the same as or differ from each other. There is no particular limitation on positions of substituents. Examples of substituents are the same as those described as examples of substituents of the aliphatic hydrocarbon group.

X^{a} is a halogen atom, which is specifically a chlorine atom, a bromine atom, an iodine atom, or a fluorine atom, and preferably a chlorine atom. The use of an inexpensive organic chloride as the starting compound is more advantageous from economic standpoints. Furthermore, the use of an organic chloride does not pose problems such as localized production areas and poor availability of bromine, which occur when an organic bromide is used, bromine being a raw material of the organic bromide, and does not pose problems such as the need for waste treatment facilities that pose a large load in industrialization.

Accordingly, chlorobenzene is particularly preferable as the organic halide represented by General Formula I (R^{a}-X^{a}), which is the starting material. A commercially available organic halide or an organic halide produced using a method known in the art may be used.

The dispersion product (hereinafter may be abbreviated as "SD" which is an abbreviation of Sodium Dispersion) obtained by dispersing sodium in a dispersion solvent is obtained by dispersing minute particles of sodium in an antisolvent or dispersing sodium in a liquid form in an antisolvent. Examples of sodium include sodium metal and an alloy containing sodium metal. An average particle diameter of minute particles is preferably smaller than 10 µm, and minute particles having an average particle diameter smaller than 5 µm can be particularly preferably used. The average particle diameter indicates the diameter of a sphere that has a projected area equivalent to a projected area obtained through image analysis of a micrograph.

A solvent known in the art can be used as the dispersion solvent so long as minute particles of sodium or sodium in a liquid form can be dispersed in an antisolvent and the solvent does not inhibit a reaction between the organic halide, which is the starting compound, and the SD. Examples of the dispersion solvent include aromatic solvents such as xylene and toluene, normal paraffin-based solvents such as normal decane, heterocyclic compound solvents such as tetrahydrothiophene, and a solvent obtained by mixing any of these.

It is preferable to use an SD that has such activity that if 2.1 or more molar equivalents of the SD is reacted with chlorobenzene in a reaction solvent such as hexane, the yield of phenylsodium is 99.0% or more with respect to added chlorobenzene. If such a highly active SD is used, an organic magnesium compound can be more efficiently synthesized. To keep the activity of the SD high, the SD is preferably stored in a container, such as a glass vial, that has good gas barrier properties. However, the present disclosure does not exclude a case in which the SD is stored in a container that has poor gas barrier properties, and in such a case, the SD is used immediately after being manufactured, e.g., within a few weeks, and preferably within three weeks.

A solvent known in the art can be used as the reaction solvent in step 1 so long as the solvent does not inhibit the reaction between the organic halide, which is the starting compound, and the SD. Specifically, a known solvent that has low reactivity with the organic halide and the SD and low polarity can be used. For example, paraffin-based solvents such as normal paraffin-based solvents and cycloparaffin-based solvents can be particularly preferably used. The use of ether-based solvents, amine-based solvents, heterocyclic compound solvents, benzene, which is an aromatic solvent, and the like is not excluded, but if solvents that have relatively high reactivity with the organic halide and the SD are used as the reaction solvent, the reaction temperature needs to be controlled to be around 0°C, for example. Normal hexane, normal pentane, normal heptane, normal decane, cyclohexane, and the like are preferable as paraffin-based solvents, and in particular, normal hexane, which is inexpensive and can be easily collected through distillation due to its low boiling point, can be preferably used. A cyclic ether solvent is preferable as an ether-based solvent, and tetrahydrofuran or the like can be preferably used. Ethylenediamine or the like can be preferably used as an amine-based solvent. Tetrahydrothiophene or the like can be used as a heterocyclic compound solvent. It is possible to use only one of these solvents or a combination of two or more of these solvents as a mixed solvent. The reaction solvent may be of the same type as the above-described dispersion solvent or a different type from the dispersion solvent.

If a paraffin-based solvent is used as the reaction solvent, there is no particular limitation on a reaction temperature in step 1, and the reaction temperature can be appropriately set according to types and amounts of the organic halide, which is the starting compound, the SD, and the reaction solvent, a reaction pressure, and the like. Specifically, the reaction temperature is preferably set so as not to exceed the boiling point of the reaction solvent. In a pressurized state, the boiling point is higher than that under atmospheric pressure, and accordingly the reaction temperature can be set to a high temperature. The reaction can also be carried out at room temperature, and the reaction temperature is preferably 0 to 100°C, particularly preferably 20 to 80°C, further preferably from room temperature to 60°C, more preferably 40 to 60°C, and yet more preferably 40 to 50°C. An excessively low reaction temperature reduces the reaction rate and therefore is not preferable, and an excessively high reaction temperature causes a Wurtz reaction and therefore is not preferable. A special temperature control means for heating, cooling, and the like need not be provided, but a temperature control means may also be provided as necessary. On the other hand, if a solvent other than a paraffin-based solvent is used as the reaction solvent, the reaction is preferably carried out at a low temperature, preferably around 0°C to prevent a reaction between the reaction solvent and phenylsodium, which is a preferable example of the compound represented by General Formula II (R^{a}-Na), which is produced in the reaction in step 1, for example. In cases such as one in which chlorobenzene, which is a preferable example of the organic halide represented by General Formula I (R^{a}-X^{a}), is used, if THF is added in an equimolar amount relative to chlorobenzene, generation of biphenyl can be effectively suppressed and a favorable reaction rate can be maintained. Accordingly, a target compound can be efficiently synthesized as a result of the type and the amount of the reaction solvent being appropriately controlled.

There is no particular limitation on a reaction time of step 1, and the reaction time can be appropriately set according to types and amounts of the organic halide, which is the starting compound, the SD, and the reaction solvent, the reaction pressure, the reaction temperature, and the like. Usually, the reaction is carried out for 15 minutes to 24 hours, and preferably for 20 minutes to 6 hours.

There is no particular limitation on the amount of the SD added in step 1, and the amount of the SD can be appropriately set according to types and amounts of the organic halide, which is the starting compound, the SD, and the reaction solvent, the reaction pressure, the reaction temperature, and the like. However, if the amount of the SD is smaller than 2.0 molar equivalents relative to the organic halide, which is the starting material, a Wurtz reaction in which starting materials are coupled to each other occurs, for example, and therefore the amount of the SD is preferably at least 2.0 molar equivalents and not larger than 2.5 molar equivalents.

The SD and reagents such as the reaction solvent can be stably handled under atmospheric pressure, and therefore normal pressure conditions under atmospheric pressure are suitable for step 1. However, phenylsodium, which is a preferable example of R^{a}-Na to be produced, is highly active and protonated with moisture if even a small amount of air is mixed in, and therefore step 1 may also be carried out in an inert gas atmosphere filled with an argon gas, a nitrogen gas, or the like, as necessary.

The organic sodium compound obtained through step 1 and represented by General Formula II (R^{a}-Na) is obtained as a result of the halogen atom of the organic halide represented by General Formula I (R^{a}-X^{a}), which is the starting compound, being substituted by sodium. Accordingly, in General Formula II (R^{a}-Na), R^{a} is the same as R^{a} in the above-described General Formula I, and Na is a sodium atom.

The obtained organic sodium compound may also be purified using a purification means known in the art, such as column chromatography, distillation, or recrystallization. Also, the organic halide that is the starting compound and is left unreacted may be collected and again used for the reaction in step 1. This may also be carried out in an inert gas atmosphere filled with an argon gas, a nitrogen gas, or the like, as is the case with the generation of the organic sodium compound.

### Step 2

As expressed by Reaction Formula II (R^{a}-Na + Mg-(X^{b})₂ → R^{a}-Mg-X^{b} + NaX^{b}), step 2 is a step for reacting the organic sodium compound obtained in step 1 and represented by General Formula II (R^{a}-Na) with a magnesium halide represented by General Formula III (Mg-(X^{b})₂) to obtain an organic magnesium compound represented by General Formula IV (R^{a}-Mg-X^{b}) as a final target compound.

In the magnesium halide represented by General Formula III (Mg-(X^{b})₂), X^{b} is a halogen atom, specifically, a chlorine atom, a bromine atom, an iodine atom, or a fluorine atom, and magnesium chloride, magnesium bromide, or magnesium iodide can be preferably used as the magnesium halide represented by General Formula III (Mg-(X^{b})₂). Anhydrous magnesium chloride is particularly preferable. The use of anhydrous magnesium chloride, which is easily available and inexpensive, is extremely advantageous in economic and industrial standpoints. Furthermore, the use of anhydrous magnesium chloride is advantageous in that an organic magnesium compound that is highly reactive in a Grignard reaction can be obtained.

A commercially available magnesium halide or a magnesium halide produced using a method known in the art may be used.

If magnesium metal is used, there is a problem in that magnesium metal is deposited on a bottom portion of a reaction vessel and accordingly, the reaction locally progresses and is difficult to control, and scaling up the reaction is difficult in terms of safety. On the other hand, an anhydrous magnesium halide can be more easily formed into fine powder than magnesium metal, and if the anhydrous magnesium halide is formed into fine powder, the reaction progresses within the entire reaction vessel. Accordingly, the reaction moderately and smoothly progresses and can be safely scaled up.

Step 2 may be carried out by adding the magnesium halide to a reaction product obtained through step 1 or adding the magnesium halide in the presence of a reaction solvent after purifying the reaction product obtained through step 1 using a purification means known in the art. Reaction solvents that can be used are the same as those that can be used in step 1.

If a paraffin-based solvent is used as the reaction solvent, there is no particular limitation on a reaction temperature in step 2, and the reaction temperature can be appropriately set according to types and amounts of the organic sodium compound, the SD, and the reaction solvent, a reaction pressure, and the like. Specifically, the reaction temperature is preferably set so as not to exceed the boiling point of the reaction solvent. In a pressurized state, the boiling point is higher than that under atmospheric pressure, and accordingly the reaction temperature can be set to a high temperature. The reaction can also be carried out at room temperature, and the reaction temperature is preferably 0 to 100°C, particularly preferably 20 to 80°C, further preferably from room temperature to 60°C, more preferably 40 to 60°C, and yet more preferably 40 to 50°C. A special temperature control means for heating, cooling, and the like need not be provided, but a temperature control means may also be provided as necessary. On the other hand, if a solvent other than a paraffin-based solvent is used as the reaction solvent, the reaction is preferably carried out at a low temperature, preferably around 0°C to prevent a reaction between the reaction solvent and phenylsodium, which is a preferable example of R^{a}-Na produced in the reaction in step 1, for example.

There is no particular limitation on a reaction time of step 2, and the reaction time can be appropriately set according to types and amounts of the organic sodium compound, the SD, and the reaction solvent, the reaction pressure, the reaction temperature, and the like. Usually, the reaction is carried out for 5 minutes to 2 hours, and preferably for 10 minutes to 1 hour.

The magnesium halide and reagents such as the reaction solvent can be stably handled under atmospheric pressure, and therefore normal pressure conditions under atmospheric pressure are suitable for step 2. However, step 2 may also be carried out in an inert gas atmosphere filled with an argon gas, a nitrogen gas, or the like, as necessary according to the type of the organic sodium compound and the like.

Through the reaction in step 2, an organic magnesium compound represented by General Formula IV (R^{a}-Mg-X^{b}) can be obtained as the desired final target compound. As a result of a reaction R^{a}-Na + Mg-(X^{b})₂ → R^{a}-Mg-X^{b} +NaX^{b} or 2R^{a}-Na + Mg-(X^{b})₂ → R^{a}-Mg-R^{a} + 2NaX^{b} occurring in step 2, R^{a}-Mg-X^{b} or R^{a}-Mg-R^{a} can be obtained. The organic magnesium represented by General Formula IV (R^{a}-Mg-X^{b}) synthesized in this step is obtained as a result of sodium of the organic sodium compound represented by General Formula II (R^{a}-Na) being substituted by a magnesium halide or magnesium. Accordingly, in General Formula IV (R^{a}-Mg-X^{b}), R^{a} is the same as R^{a} in the above-described General Formula I (R^{a}-X^{a}) and General Formula II (R^{a}-Na), and if X^{b} is a halogen, X^{b} is derived from the magnesium halide and is the same as X^{b} in General Formula III (Mg-(X^{b})₂).

The organic magnesium compound finally synthesized in the method for synthesizing an organic magnesium compound according to the present embodiment may also be purified using a purification means known in the art, such as recrystallization. Also, the organic halide, the organic sodium compound, and the like that are left unreacted may be collected and again used for the synthesis of the organic magnesium compound.

### Method for Synthesizing Organic Boronic Acid Compound

In the method for synthesizing an organic boronic acid compound according to the present embodiment, the organic magnesium compound obtained using the method for synthesizing an organic magnesium compound according to the present embodiment is reacted with a borate ester compound represented by General Formula V (B-(O-R^{b})₃) to obtain an organic boronic acid compound represented by General Formula VI (R^{a}-B(OH)₂) (step 3A).

### Step 3 A

As expressed by Reaction Formula IIIa (R^{a}-Mg-X^{b} + B-(O-R^{b})₃ → R^{a}-B-(O-R^{b})₂ + Mg-X^{b}(O-R^{b})) and Reaction Formula IIIb (R^{a}-B-(O-R^{b})₂ + 2H₂O → R^{a}-B(OH)₂ + 2R^{b}-OH), step 3A is a step for reacting the organic magnesium compound obtained through the above-described steps 1 and 2 performed using the SD with a borate ester compound represented by General Formula V (B-(O-R^{b})₃) to obtain an organic boronic acid compound represented by General Formula VI (R^{a}-B(OH)₂). This is a step for synthesizing an organic boronic acid compound to be used as a coupling target compound in Suzuki-Miyaura coupling and the like, by using an organic magnesium compound synthesized using the SD and represented by a Grignard reagent.

In the borate ester compound represented by General Formula V (B-(O-R^{b})₃), R^{b} is an aliphatic hydrocarbon group, an alicyclic hydrocarbon group, or an aromatic hydrocarbon group, which optionally includes a substituent.

The aliphatic hydrocarbon group, the alicyclic hydrocarbon group, and the aromatic hydrocarbon group that optionally include substituents are as described above, and R^{b} is preferably an isopropyl group, a methyl group, an ethyl group, or the like, and particularly preferably an isopropyl group.

Accordingly, triisopropyl borate can be preferably used as the borate ester compound represented by General Formula V (B-(O-R^{b})₃). A commercially available borate ester compound or a borate ester compound produced using a method known in the art may be used.

Step 3A may be carried out by adding the borate ester compound to a reaction product obtained through step 2 or adding the borate ester compound in the presence of a reaction solvent after purifying the reaction product obtained through step 2 using a purification means known in the art. Reaction solvents that can be used are the same as those that can be used in steps 1 and 2, and the reaction in step 3A needs to be performed in an anhydrous solvent.

If a paraffin-based solvent is used, there is no particular limitation on a reaction temperature in step 3A, and the reaction temperature can be appropriately set according to types and amounts of the organic magnesium compound, the borate ester compound, and the reaction solvent, a reaction pressure, and the like. Specifically, the reaction temperature is preferably set so as not to exceed the boiling point of the reaction solvent. In a pressurized state, the boiling point is higher than that under atmospheric pressure, and accordingly the reaction temperature can be set to a high temperature. The reaction can also be carried out at room temperature, and the reaction temperature is preferably 0 to 100°C, particularly preferably 20 to 80°C, further preferably from room temperature to 60°C, more preferably 40 to 60°C, and yet more preferably 40 to 50°C. A special temperature control means for heating, cooling, and the like need not be provided, but a temperature control means may also be provided as necessary.

There is no particular limitation on a reaction time of step 3A, and the reaction time can be appropriately set according to types and amounts of the organic magnesium compound, the borate ester compound, and the reaction solvent, the reaction pressure, the reaction temperature, and the like. Usually, the reaction is carried out for 5 minutes to 2 hours, and preferably for 10 minutes to 1 hour.

The borate ester compound and reagents such as the reaction solvent can be stably handled under atmospheric pressure, and therefore normal pressure conditions under atmospheric pressure are suitable for step 3A. However, step 3A may also be carried out in an inert gas atmosphere filled with an argon gas, a nitrogen gas, or the like, as necessary.

Through the reaction in step 3A, the organic boronic acid compound represented by General Formula VI (R^{a}-B(OH)₂) can be obtained as a desired final target compound. Specifically, in the synthesis of the organic boronic acid compound represented by General Formula VI (R^{a}-B(OH)₂) through the reaction in step 3A, the organic magnesium compound represented by General Formula IV (R^{a}-Mg-X^{b}) is reacted with the borate ester compound represented by General Formula V (B-(O-R^{b})₃) to synthesize a boronate ester compound represented by General Formula VII (R^{a}-B-(O-R^{b})₂). As a result of the boronate ester compound being hydrolyzed, the organic boronic acid compound represented by General Formula VI (R^{a}-B(OH)₂) can be obtained, and in this process, the organic boronic acid compound is synthesized in an anhydrous solvent, and accordingly forms a cyclic anhydride (boroxine) through dehydrative trimerization.

The organic boronic acid compound represented by General Formula VI (R^{a}-B(OH)₂) is obtained as a result of a magnesium halide group of the organic magnesium compound represented by General Formula IV (R^{a}-Mg-X^{b}) being substituted by a boronic acid group. Accordingly, in General Formula VI (R^{a}-B(OH)₂), R^{a} is the same as R^{a} in the above-described General Formula I (R^{a}-X^{a}), General Formula II (R^{a}-Na), and General Formula IV (R^{a}-Mg-X^{b}).

The organic boronic acid compound represented by General Formula VI (R^{a}-B(OH)₂) is obtained as a result of the magnesium halide group (-Mg-X^{b} group) of the organic magnesium compound represented by General Formula IV (R^{a}-Mg-X^{b}) being substituted by the boronic acid group (-B(OH)₂ group). Accordingly, in General Formula VI (R^{a}-B(OH)₂), R^{a} is the same as R^{a} in the above-described General Formula I (R^{a}-X^{a}), General Formula II (R^{a}-Na), and General Formula IV (R^{a}-Mg-X^{b}).

The organic boronic acid compound finally synthesized in the method for synthesizing an organic boronic acid compound according to the present embodiment may also be purified using a purification means known in the art, such as recrystallization. Also, the organic halide, the organic sodium compound, the organic magnesium compound, the magnesium halide, the borate ester compound, and the like that are left unreacted may be collected and again used for the synthesis of the organic magnesium compound and the organic boronic acid compound.

### Coupling Reaction

In a coupling reaction according to the present embodiment, the organic magnesium compound obtained using the method for synthesizing an organic magnesium compound according to the present embodiment is reacted with an organic halide represented by General Formula VIII (R^{c}-X^{c}) in the presence of an iron catalyst, a nickel catalyst, a palladium catalyst, a chromium catalyst, a manganese catalyst, a cobalt catalyst, or a copper catalyst to obtain a coupling compound represented by General Formula IX (R^{a}-R^{c}) (step 3B).

### Step 3B

As expressed by Reaction Formula IV (R^{a}-Mg-X^{b} + R^{c}-X^{c} → R^{a}-R^{c} + Mg-X^{b}X^{c}), step 3B is a step for reacting the organic magnesium compound obtained through the above-described steps 1 and 2 performed using the SD with an organic halide represented by General Formula VIII (R^{c}-X^{c}) in the presence of an iron catalyst, a nickel catalyst, a palladium catalyst, a chromium catalyst, a manganese catalyst, a cobalt catalyst, or a copper catalyst to obtain a coupling compound represented by General Formula IX (R^{a}-R^{c}). That is, the organic magnesium compound synthesized using the SD and represented by a Grignard reagent is used for a Kumada-Tamao-Corriu coupling reaction.

In the organic halide represented by General Formula VIII (R^{c}-X^{c}), R^{c} is an aromatic hydrocarbon group or an allyl group, which optionally includes a substituent.

The aromatic hydrocarbon group optionally including a substituent is as described above. In the allyl group optionally including a substituent, the allyl group is a monovalent unsaturated hydrocarbon group CH₂=CH-CH₂- that is obtained by removing one hydrogen atom from a methyl group in propylene CH₂=CH-CH₃. The substituent is as described above.

X^{c} is a halogen atom, specifically, a chlorine atom, a bromine atom, an iodine atom, or a fluorine atom.

A commercially available organic halide or an organic halide produced using a method known in the art may be used as the organic halide represented by General Formula VIII (R^{c}-X^{c}).

There is no particular limitation on the iron catalyst, the nickel catalyst, the palladium catalyst, the chromium catalyst, the manganese catalyst, the cobalt catalyst, and the copper catalyst so long as the catalysts can be used for the Kumada-Tamao-Corriu coupling reaction, and known catalysts can be used. Examples of catalysts that can be used include iron catalysts such as FeF₃·3H₂O, FeF₂·4H₂O, FeF₃, FeCl₃, and Fe(acac)₃, nickel catalysts such as NiCl₂(dppp), Ni(acac)₂, Ni(MeCN)₂Cl₂, NiF₂, NiF₂·4H₂O, NiCh, and MCl₂·6H₂O, palladium catalysts such as Pd(PPh₃)₄, Pd(OAc)₂, and Pd(MeCN)₂Cl₂, chromium catalysts such as CrCl₂, manganese catalysts such as MnCh, cobalt catalysts such as CoF₂·H₂O, CoF₂·4H₂O, CoF₂, CoF₃, and CoCl₂·6H₂O, and copper catalysts such as CuCl₂, Cul, CuBr, CuTc, and Cu(OTf)₂.

Step 3B may be carried out by adding the organic halide to a reaction product obtained through step 2 or adding the organic halide in the presence of a reaction solvent after purifying the reaction product obtained through step 2 using a purification means known in the art. Reaction solvents that can be used are the same as those that can be used in steps 1 and 2, and the reaction in step 3B is preferably performed in an anhydrous solvent.

If a paraffin-based solvent is used, there is no particular limitation on a reaction temperature in step 3B, and the reaction temperature can be appropriately set according to types and amounts of the organic magnesium compound, the organic halide, and the reaction solvent, a reaction pressure, and the like. Specifically, the reaction temperature is preferably set so as not to exceed the boiling point of the reaction solvent. In a pressurized state, the boiling point is higher than that under atmospheric pressure, and accordingly the reaction temperature can be set to a high temperature. The reaction can also be carried out at room temperature, and the reaction temperature is preferably 0 to 100°C, particularly preferably 20 to 80°C, further preferably from room temperature to 60°C, more preferably 40 to 60°C, and yet more preferably 40 to 50°C. A special temperature control means for heating, cooling, and the like need not be provided, but a temperature control means may also be provided as necessary.

There is no particular limitation on a reaction time of step 3B, and the reaction time can be appropriately set according to types and amounts of the organic magnesium compound, the organic halide, and the reaction solvent, the reaction pressure, the reaction temperature, and the like. Usually, the reaction is carried out for 5 minutes to 2 hours, and preferably for 10 minutes to 1 hour.

An inert gas atmosphere filled with an argon gas, a nitrogen gas, or the like is suitable for step 3B.

Through the reaction in step 3B, the coupling compound represented by General Formula IX (R^{a}-R^{c}) can be obtained as a desired final target compound. The coupling compound represented by General Formula IX (R^{a}-R^{c}) is obtained as a result of an organic group (-R^{a} group) of the organic magnesium compound represented by General Formula IV (R^{a}-Mg-X^{b}) and an organic group (-R^{c} group) of the organic halide represented by General Formula VIII (R^{c}-X^{c}) being linked to each other. Accordingly, in General Formula IX (R^{a}-R^{c}), R^{a} is the same as R^{a} in the above-described General Formula I (R^{a}-X^{a}), General Formula II (R^{a}-Na), and General Formula IV (R^{a}-Mg-X^{b}), and R^{c} is the same as R^{c} in the above-described General Formula VIII (R^{c}-X^{c}).

The coupling compound finally synthesized in the coupling method according to the present embodiment may also be purified using a purification means known in the art, such as recrystallization. Also, the organic halide, the organic sodium compound, the organic magnesium compound, the magnesium halide, and the like that are left unreacted may be collected and again used for the synthesis of the organic magnesium compound and the coupling reaction.

With the method for synthesizing an organic magnesium compound according to the present embodiment, an organic magnesium compound can be stably and efficiently synthesized using the SD. As a result of the SD, which can be easily handled, being used, the organic magnesium compound can be easily synthesized under moderate conditions at a low cost in a short time with few steps that do not involve a complex chemical method, and therefore the synthesis method is extremely advantageous from economic and industrial standpoints. Sodium is extremely widely distributed on the earth, and therefore this technology has excellent sustainability. On the other hand, if sodium metal in a solid form is used, the efficiency is low if the reaction temperature is room temperature, and therefore the reaction needs to be carried out at a high temperature, e.g., at least a melting point (98°C) of sodium metal. If the reaction is carried out at such a high temperature, a Wurtz reaction in which organic halides are coupled to each other occurs, and accordingly the organic sodium compound cannot be efficiently synthesized and the yield of the organic magnesium compound is reduced. In contrast, if the SD is used, the reaction can progress under moderate conditions, and therefore the organic sodium compound can be efficiently obtained without a side reaction such as the Wurtz reaction being caused, and consequently the organic magnesium compound can be synthesized with a high yield and high purity. Furthermore, the method for synthesizing an organic magnesium compound according to the present embodiment can be favorably used for the preparation of an aromatic Grignard reagent, in particular, an aromatic Grignard reagent including a bulky substituent, and an alkenyl Grignard reagent, which have been difficult to prepare.

The organic magnesium compound synthesized using the method for synthesizing an organic magnesium compound according to the present embodiment can be favorably used for Suzuki-Miyaura coupling, Kumada-Tamao-Corriu coupling, and the like. Accordingly, the method for synthesizing an organic magnesium compound according to the present embodiment can be used in various technical fields such as natural product total synthesis and the synthesis of functional materials such as medicines, agricultural chemicals, and electronic materials.

Furthermore, the organic magnesium compound that is stably and efficiently synthesized using the SD in the method for synthesizing an organic magnesium compound according to the present embodiment is used in the method for synthesizing an organic boronic acid compound according to the present embodiment. Therefore, an organic boronic acid compound that can be favorably used for Suzuki-Miyaura cross coupling and the like can be efficiently synthesized. Accordingly, the method for synthesizing an organic boronic acid compound according to the present embodiment can be used in various technical fields such as natural product total synthesis and the synthesis of functional materials such as medicines, agricultural chemicals, and electronic materials.

Also, in the coupling method according to the present embodiment, the organic magnesium compound synthesized using the method for synthesizing an organic magnesium compound according to the present embodiment is used for Kumada-Tamao-Corriu coupling. That is, in the coupling method according to the present embodiment, a coupling reaction is carried out using the organic magnesium compound stably and efficiently synthesized using the SD. Therefore, the coupling reaction can be efficiently made to progress. Accordingly, the coupling method according to the present embodiment can be used in various technical fields such as natural product total synthesis and the synthesis of functional materials such as medicines, agricultural chemicals, and electronic materials.

### Examples

Hereinafter, the present embodiment will be specifically described by use of examples, but the present embodiment is not limited to these examples. Note that in the following examples, a dispersion product obtained by dispersing minute particles of sodium metal in normal paraffin oil was used as the SD, and the substance amount of the SD was a value in terms of sodium metal contained in the SD.

### Preliminary Investigation Example 1: Investigation of Conditions for Synthesizing Organic Boronic Acid Compound

In Preliminary Investigation Example 1, the synthesis of an organic boronic acid compound was attempted by reacting an organic halide with a borate ester compound. Specifically, the synthesis of phenylboronic acid 6 as an organic boronic acid compound was investigated under synthesis conditions summarized in scheme 1 in FIG. 1.

2 mmol of chlorobenzene 1, which was an organic halide used as a starting compound, and 2.1 molar equivalents of the SD were added to 2 ml of hexane and a reaction was carried out at 25°C for 15 minutes to synthesize phenylsodium 2 as an organic sodium compound. Here, the molar equivalent value of the SD was a value relative to chlorobenzene 1 used as the starting compound. Subsequently, 2.0 molar equivalents of triisopropyl borate 4 was added as a borate ester compound, and reacted with phenylsodium 2 synthesized as described above. Here, the molar equivalent value of triisopropyl borate 4 was a value relative to chlorobenzene. After the reaction was carried out for 10 minutes, 1.0 mol/L of an aqueous hydrochloric acid solution was added to cause hydrolysis, and the reaction liquid was extracted using diethyl ether and measured using GC-MS. Through the GC-MS measurement, it was confirmed that terphenyl 7 was generated as a main compound and triphenylboron 8 was generated. However, phenylboronic acid 6, which was the target compound, could not be obtained.

Further, rather than the borate ester compound, boric acid was reacted with phenylsodium 2, the organic sodium compound, but the reaction did not progress.

### Example 1: Investigation of Conditions for Synthesizing Organic Magnesium Compound and Conditions for Synthesizing Organic Boronic Acid Compound Using Organic Magnesium Compound

In this example, the synthesis of an organic boronic acid compound was attempted by synthesizing an organic sodium compound using an organic halide as a starting material and using the SD, synthesizing an organic magnesium compound using a magnesium halide, and reacting the organic magnesium compound with a borate ester compound. Specifically, the synthesis of phenylmagnesiumchloride 3 as an organic magnesium compound was investigated under synthesis conditions summarized in scheme 2 in FIG. 1, and the synthesis of phenylboronic acid 6 as an organic boronic acid compound was investigated using obtained phenylmagnesiumchloride 3.

### Synthesis of Phenylmagnesiumchloride 3

2 mmol of chlorobenzene 1, which was an organic halide used as a starting compound, and 2.1 molar equivalents of the SD were added to 2 ml of hexane and a reaction was carried out at 60°C for 15 minutes to synthesize phenylsodium 2 as an organic sodium compound. Here, the molar equivalent value of the SD was a value relative to chlorobenzene 1 used as the starting compound. 2.1 molar equivalents of anhydrous magnesium chloride was added to phenylsodium 2 and a reaction was carried out at 25°C for 15 minutes to obtain phenylmagnesiumchloride 3 as an organic magnesium compound.

### Synthesis of Phenylboronic Acid 6

The reaction liquid obtained in the above-described synthesis was cooled to 0°C, and then 2.0 molar equivalents of triisopropyl borate 4 was added as a borate ester compound and reacted with phenylmagnesiumchloride 3 synthesized as described above. Here, the molar equivalent value of triisopropyl borate 4 was a value relative to chlorobenzene. After the reaction was carried out for 10 minutes, 1.0 mol/L of an aqueous hydrochloric acid solution was added to cause hydrolysis, and the reaction liquid was extracted using diethyl ether and measured using GC-MS. Triphenylboroxine 5 was detected in the GC-MS measurement, and this is supposed to indicate that generated organic boronic acid, phenylboronic acid 6, formed boroxine and detected in an equilibrium state. Note that a white solid was precipitated during hydrolysis. The white solid was dissolved in water; therefore, the solid was found to be sodium chloride.

### Example 2: Investigation of Conditions for Synthesizing Organic Magnesium Compound and Application to Kumada-Tamao-Corriu Coupling

In this example, an organic magnesium compound was synthesized under synthesis conditions summarized in FIG. 2, and whether or not the obtained organic magnesium compound could be used for Kumada-Tamao-Corriu coupling performed using a nickel catalyst was investigated.

The synthesis of 4-methyl-phenylmagnesiumchloride 3 as an organic magnesium compound was investigated. 1.4 molar equivalents of 4-methyl-chlorobenzene 1 as a starting compound and 3.1 molar equivalents of the SD were added to 1.4 ml of hexane, and a reaction was carried out at 25°C for 1 hour to synthesize 4-methyl-phenylsodium 2. 1.4 molar equivalents of magnesium chloride was added to the obtained 4-methyl-phenylsodium 2, and a reaction was carried out at 0°C for 1 hour in the presence of 1.4 mL of THF to obtain 4-methyl-phenylmagnesiumchloride 3.

Subsequently, whether or not the obtained 4-methyl-phenylmagnesiumchloride 3 could be used for Kumada-Tamao-Corriu coupling performed using a nickel catalyst was investigated. 4-methyl-phenylmagnesiumchloride 3 was reacted with 1.0 molar equivalent (0.5 mmol) of 2-bromonaphthalene 4 at 25°C for 24 hours in the presence of 5 mol% (relative to 2-bromonaphthalene) of dichloro[1,3-bis(diphenylphosphino)propane]nickel(II) (NiCl₂(dppp)) used as a nickel catalyst. As a result, 2-(4-methylphenyl)naphthalene 5 was obtained as shown in FIG. 2.

The synthesized 2-(4-methylphenyl)naphthalene 5 was evaluated through 1H NMR measurement. The yield was calculated as a percentage of an amount of actually obtained 2-(4-methylphenyl)naphthalene 5 relative to an amount of 2-(4-methylphenyl)naphthalene 5 that can be theoretically produced from 2-bromonaphthalene 4 added to the reaction system. The yield was 94%. Note that the isolation yield was 88%. Thus, 4-methyl-phenylmagnesiumchloride 3 could be synthesized with a high yield from 4-methyl-chlorobenzene 1 through the synthesis of 4-methyl-phenylsodium 2. Moreover, it can be understood that Kumada-Tamao-Corriu coupling performed using 4-methyl-phenylmagnesiumchloride 3 progressed efficiently, and as a result, 2-(4-methylphenyl)naphthalene 5 could be obtained with a high yield.

### Industrial Applicability

The present invention is useful in all technical fields in which the method for synthesizing an organic magnesium compound represented by a Grignard reagent and organic magnesium compounds synthesized using the synthesis method are used. In particular, the present invention can be favorably used as an intermediate product in a coupling reaction, and is particularly useful in the fields of manufacture of medicines, agricultural chemicals, and electronic materials.

## Claims

1. A method for synthesizing an organic magnesium compound, comprising:
in a reaction solvent, reacting an organic halide represented by General Formula I (R^{a}-X^{a}), where R^{a} is an aliphatic hydrocarbon group, an alicyclic hydrocarbon group, or an aromatic hydrocarbon group, which optionally includes a substituent that does not react with sodium, and X^{a} is a halogen atom, with a dispersion product obtained by dispersing sodium in a dispersion solvent to obtain an organic sodium compound represented by General Formula II (R^{a}-Na), where R^{a} is the same as R^{a} in the General Formula I; and
reacting the obtained organic sodium compound with a magnesium halide represented by General Formula III (Mg-(X^{b})₂), where X^{b} is a halogen atom, to obtain an organic magnesium compound represented by General Formula IV (R^{a}-Mg-X^{b}), where R^{a} is the same as R^{a} in the General Formula I and X^{b} is the same as X^{b} in the General Formula III or R^{a}.

2. A method for synthesizing an organic boronic acid compound, comprising
reacting the organic magnesium compound according to claim 1 with a borate ester compound represented by General Formula V (B-(O-R^{b})₃), where R^{b} is an aliphatic hydrocarbon group, an alicyclic hydrocarbon group, or an aromatic hydrocarbon group, which optionally includes a substituent, to obtain an organic boronic acid compound represented by General Formula VI (R^{a}-B(OH)₂), where R^{a} is the same as R^{a} in the General Formula I.

3. A coupling method comprising
reacting the organic magnesium compound according to claim 1 with an organic halide represented by General Formula VIII (R^{c}-X^{c}), where R^{c} is an aromatic hydrocarbon group or an allyl group, which optionally includes a substituent, and X^{c} is a halogen atom, in the presence of an iron catalyst, a nickel catalyst, a palladium catalyst, a chromium catalyst, a manganese catalyst, a cobalt catalyst, or a copper catalyst to obtain a coupling compound represented by General Formula IX (R^{a}-R^{c}), where R^{a} is the same as R^{a} in the General Formula I and R^{c} is the same as R^{c} in the General Formula VIII.
